# EUROPÄISCHE PATENTSCHRIFT

(11) **EP 1 335 666 B1**
(45) Veröffentlichungstag und Bekanntmachung des Hinweises auf die Patenterteilung: **03.01.2007**
(21) Anmeldenummer: 01997251.2
(22) Anmeldetag: 23.11.2001
(51) Int. Cl.: A61B 5/00

(54) **SENSOR UND VERFAHREN ZUM MESSEN PHYSIOLOGISCHER PARAMETER**
SENSOR AND METHOD FOR MEASURING PHYSIOLOGICAL PARAMETERS
CAPTEUR ET PROCEDE POUR LA MESURE DE PARAMETRES PHYSIOLOGIQUES

(30) Priorität: 23.11.2000 WO PCT/CH00/00628; 14.02.2001 WO PCT/CH01/00098
(43) Veröffentlichungstag der Anmeldung: 20.08.2003
(73) Patentinhaber: SENTEC AG, 4106 Therwil (CH)
(72) Erfinder: TSCHUPP, Andres, CH-4106 Therwil (CH); LANG, Joseph, F-68220 Ranspach le Haut (FR); ROOST, Matthias, CH-8239 Dörflingen (CH)
(74) Vertreter: Dr. Graf & Partner
(86) Internationale Anmeldenummer: PCT/CH2001/000685
(87) Internationale Veröffentlichungsnummer: WO 2002/041770

(56) Entgegenhaltungen:
- EP-A- 0 770 349
- WO-A-00/42911
- WO-A-00/53082
- WO-A-99/13765
- CH-A- 690 061
- US-A- 4 129 125
- US-A- 4 840 179
- US-A- 5 259 381
- US-A- 5 342 498
- US-A- 5 673 692
- US-A- 5 749 365
- US-A- 5 862 803
- US-A- 5 873 821

## Beschreibung

Die Erfindung betrifft einen Sensor zum Messen physiologischer Parameter Die Erfindung betrifft weiter ein Verfahren und ein System zum Messen physiologischer Parameter.

Die Messung eines oder mehrerer physiologischer Parameter des menschlichen Körpers gewinnt zunehmend an Bedeutung. So ist aus der Druckschrift EP 0 267 978 A1 ein Kombinationssensor zur kombinierten Messung der Sauerstoffsättigung des Hämoglobins im arteriellen Blut sowie des arteriellen Kohlendioxidpartialdruckes bekannt. Zur Messung der Sauerstoffsättigung (SpO₂) wird eine nichtinvasive, optische, allgemein bekannte Methode, welche als Pulsoximetrie bezeichnet wird, verwendet. Ein derartiges Pulsoximetersystem umfasst einen Sensor, welcher an einer gut durchbluteten Stelle am menschlichen Körper angelegt wird, ein Pulsoximeter, sowie ein Verbindungskabel, welches den Sensor mit dem Pulsoximeter verbindet. Zur Messung der CO₂-Konzentration im Blut wird der transkutane Kohlendioxidpartialdruck (tcpCO₂) mit Hilfe einer elektrochemischen Messvorrichtung bestimmt. Ausführliche Informationen über diese allgemein bekannten Messmethoden sind beispielsweise dem folgenden Übersichtsartikel zu entnehmen: "Noninvasive Assessment of Blood Gases, State of the Art" von J. S. Clark et al., Am. Rev. Resp. Dis., Vol. 145, 1992, pp. 220-232. Details zur pulsoximetrischen Messmethode sind beispielsweise der Druckschrift WO 00/42911 zu entnehmen.

Nachteilig an dem in der Druckschrift EP 0 267 978 A1 offenbarten Sensor sind die Tatsachen,
- dass auftretende Störsignale die Messung verfälschen,
- dass der Sensor öfters zu kalibrieren ist,
- dass der Sensor ein relativ dickes Kabel aufweist,
- dass der Sensor relativ schwer ist und einen grossen Durchmesser aufweist
- dass der Sensor zusammen mit der Auswertevorrichtung relativ teuer ist,
- und dass der Sensor nur relativ einfache Messungen durchzuführen erlaubt.

CH-A-690 061 offenbart einen Sensor zum kombinierten Messen der arteriellen Sauerstoffsättigung und des transkutanen CO₂-Partialdrucks am Ohrläppchen. Der Sensor umfasst eine Erwärmungsvorrichtung sowie Thermistoren zur Regelung und Kontrolle der gewählten Sensortemperatur, und eine elektronische Einheit, in der bereits ein Teil der Signalverarbeitung vorgenommen wird.

US-A-5 862 803 offenbart ein System zum Messen physiologischer Parameter. Die Elektroden umfassen zumindest einen Sensor und digitalen Sensorsignalprozessor, sowie Mittel zur kabellosen Übertragung der digitalen Daten mittels bidirektionalem Datenaustausch an eine Auswerteeinheit.

Es ist Aufgabe der vorliegenden Erfindung einen vorteilhafteren Sensor vorzuschlagen. Diese Aufgabe wird gelöst mit einem Sensor aufweisend die Merkmale von Anspruch 1. Die Unteransprüche 2 bis 25 betreffen weitere, vorteilhaft ausgestaltete Sensoren. Die Aufgabe wird weiter gelöst mit einem Verfahren aufweisend die Merkmale von Anspruch 26. Die Unteransprüche 27 bis 33 betreffen weitere, vorteilhafte Verfahrensschritte.

Der erfindungsgemässe Sensor weist einen innerhalb des Sensors angeordneten digitalen Sensorsignalprozessor auf, welcher das von der Messvorrichtung gemessene Signal digitalisiert, sodass dieses im Sensor zur weiteren Verarbeitung in digitaler Form vorliegt. Ein derartiger digitaler Sensorsignalprozessor wird im Englischen auch als DSSP (Digital Sensor Signal Processor) oder als Single Chip MCU (MicroComputerUnit) bezeichnet. Ein derartiger Sensorsignalprozessor umfasst auf einem einzige Chip nicht nur einen Mikrokontroller mit Speicher, Mikroprozessor und Schnittstellen, sondern auch einen Analog-Digital-Wandler sowie einen Digital-Analog-Wandler. Der digitale Sensorsignalprozessor ermöglicht unter anderem die von den im Sensor angeordneten Messvorrichtungen gemessenen Werte innerhalb des Sensors in digitale Werte zu wandeln.

Dadurch ergeben sich insbesondere folgende Vorteile:
- Das gemessene, analoge Signal wird innerhalb des Sensors in ein störungsunempfindliches digitales Signal gewandelt. Dies ermöglicht auch schwache analoge Signale sauber zu messen.
- Das Signal wird digital zwischen dem Sensor und einer nachgeordneten Auswertevorrichtung übertragen, was durch Einstreuung bedingte Signalverfälschungen weitgehend ausschliesst. Zudem ist zwischen dem Sensor und der nachgeordneten Auswertevorrichtung eine bidirektionale Kommunikation beziehungsweise ein Datenaustausch möglich.
- Die Auswertevorrichtung kann innerhalb des Sensors oder vom Sensor beabstandet angeordnet sein. In einer vorteilhaften Ausgestaltung wird die Auswertung der Signale im Sensorsignalprozessor durchgeführt.
- Zur Übertragung der digitalen Daten genügen zwei Leiterdrähte beziehungsweise ein zweipoliges Kabel. Das Verbindungskabel bestehend aus nur zwei Leiterdrähten zwischen dem Sensor und der Auswertevorrichtung kann daher sehr dünn und flexibel ausgestaltet sein. Zudem kann die Energieversorgung des Sensors über dieselben zwei Leiterdrähte erfolgen.
- In der Auswertevorrichtung wird im wesentlichen eine digitale Signalverarbeitung durchgeführt. Dies ermöglicht eine kostengünstige, als Standardprodukt erhältliche Auswertevorrichtung mit einem Prozessor und entsprechend dem Sensor angepasster Software.

In einer weiteren vorteilhaften Ausgestaltung ist der Sensor derart ausgestaltet, dass dessen digitale Ausgangssignale festgelegte, normierte Werte aufweisen. In einer bevorzugten Ausgestaltung ist im Sensor eine Referenzkurve beziehungsweise eine Eichkurve der im Sensor angeordneten Messvorrichtung gespeichert. Derartige Sensoren weisen den Vorteil auf, dass bei einem Sensoraustausch kein aufwendiges Kalibieren erforderlich ist, da alle Sensoren ein festgelegtes Ausgangssignals aufweisen.

In einer weiteren vorteilhaften Ausgestaltung weist der Sensor eine Leiterplatte auf, welche mit den wesentlichen oder allen erforderlichen elektronischen Komponenten bestückt ist. Ein derartiger Sensor ist äusserst kostengünstig herstellbar.

In einer weiteren vorteilhaften Ausgestaltung weist der Sensor einen gegen aussen im wesentlichen elektrisch abgeschirmten Innenraum auf, was den Vorteil ergibt, dass die gemessenen Signale kaum oder nicht mit Störsignalen überlagert sind.

Es ist von zentraler Bedeutung, dass der erfindungsgemässe Sensor auch schwache Signale eindeutig zu messen erlaubt, und dass die gemessenen Signale störungsfrei einer Signalauswertevorrichtung zuführbar sind. Dies hat zur Folge, dass zur Signalauswertung kein aufwendiges Verfahren erforderlich ist, um ein ansonst üblicherweise verrauschtes Signal eindeutig und reproduzierbar auszuwerten.

Als besonders vorteilhaft erweist sich die Ausgestaltung des erfindungsgemässen Sensors als ein Kombinationssensor zur kombinierten Messung der Sauerstoffsättigung des Hämoglobins im arteriellen Blut sowie des arteriellen Kohlendioxidpartialdruckes. Der in der Druckschrift EP 0 267 978 A1 offenbarte Kombinationssensor weist eine relativ grosse und entsprechend schwere Referenzelektrode aus Silber auf, welche ein stabiles Messsignal gewährleistet. Im erfindungsgemässen Kombinationssensor ist nur noch eine Referenzelektrode mit geringem Durchmesser bzw. geringer Auflagefläche erforderlich zur Messung des arteriellen Kohlendioxidpartialdruckes, da der digitale Sensorsignalprozessor in der Lage ist auch ein relativ schwaches elektrisches Signal zu verwerten, und das bereits im Sensor digitalisierte Signal störungsunempfindlich ist. Die kleine Ausgestaltung der Referenzelektrode hat zur Folge, dass der gesamte Sensor relativ klein und insbesondere auch sehr leicht ausgestaltbar ist.

In einer vorteilhaften Ausgestaltung weist der gesamte Sensor ein Gewicht von weniger als 10 Gramm auf, vorzugsweise sogar ein Gewicht von weniger als 5 Gramm.

Als besonders vorteilhaft erweist es sich den erfindungsgemässen Sensor am Ohr, insbesondere am Ohrläppchen zu verwenden. Das Ohrläppchen ist an sich ein schwieriger Messort, weil nur eine geringe Messfläche zur Verfügung steht, und weil grössere angreifende Druckkräfte die Durchblutung des Ohrläppchens stören. Der erfindungsgemässe Sensor kann mit einer zur Auflage am Ohrläppchen bestimmten Sensorauflagefläche mit entsprechend angepasstem, kleinem Durchmesser ausgestaltet sein. Zudem ist der erfindungsgemässe Sensor sehr leicht, sodass zu dessen Befestigung am Ohr nur eine geringe Klemmkraft erforderlich ist. Zudem kann das Verbindungskabel des Sensors sehr dünn und flexibel ausgestaltet sein, sodass Kopfbewegungen die Sensormessung kaum beeinflussen. Zudem ist es besonders vorteilhaft den Sensor mit einer Heizvorrichtung zu erwärmen, um dadurch das Ohrläppchen auf einer reproduzierbar konstanten Temperatur zu halten. Das Ohrläppchen erweist sich als ein besonders vorteilhafter Messort, weil sich das Ohr bezüglich Blutkreislauf relativ nahe am Herz befindet, wesentlich näher als die beispielsweise auch zur Messung von Sauerstoff oder Kohlendioxid im Blut geeignete Fingerkuppe. Zudem tritt am Ohr, auf Grund der Erwärmung, kaum eine Vasokonstruktion bzw. kaum eine Gefässverengung auf. Der erfindungsgemässe Sensor ermöglicht somit am Ohrläppchen mit einem sehr geringen Signal to Noise (S/N) Verhältnis zu messen, sodass ein Messsignal mit ausgezeichneter Qualität zur Verfügung steht.

Diese hohe Signalqualität ermöglicht nun wiederum aus den gemessenen Werten weitere physiologische Parameter zu bestimmen, wie zum Beispiel der Blutdruck, indem dieser mittels des sogenannten CNIBP-Verfahren gemessen wird, was auf Englisch "Continuous Nonlnvasive Blood Pressure" bedeutet. Dabei wird der systolische Blutdruck beispielsweise mittels der pulsoximetrischen, am Ohrläppchen messbaren Messmethode bestimmt, wie dies im Detail beispielsweise in folgender Druckschrift beschrieben ist: "Can Pulse Oximetry Be Used to Measure Systolic Blood Pressure?, R. Chawla et al., Anesth Analg, 1992; 74:196-200". Der erfindungsgemässe Sensor erlaubt die Pulskurvenform beziehungsweise das sogenannte Pletismogramm zu messen, indem zum Beispiel 50 oder 100 mal pro Sekunde der Sauerstoffgehalt des Blutes pulsoximetrisch gemessen wird, und aus dem sich ergebenden Kurvenverlauf der Blutdruck bestimmt wird.

Die hohe Signalqualität ermöglicht als weiteren physiologischen Parameter den Hematokrit, international mit "HCT" abgekürzt, zu bestimmen. Die Ermittlung dieses Parameters ist beispielsweise in der Druckschrift US 5,803,908 im Detail offenbart.

Der erfindungsgemässe Sensor ist auch zur Messung der Zusammensetzung der Atemluft geeignet.

Auf Grund der bidirektionalen Datenkommunikation zwischen dem Sensor und einer nachgelagerten Auswertevorrichtung ist in einer weiteren vorteilhaften Ausgestaltung im Sensor ein Datenspeicher angeordnet, in welchem beispielsweise gemessene Werte oder Patientendaten speicherbar sind. In einer vorteilhaften Ausgestaltung ist dieser Datenspeicher derart gross bemessen, dass über einen längeren Zeitraum gemessene Daten im Sensor speicherbar sind, beispielsweise in einem nichtflüchtigen Speicher, auch als EEPROM bezeichnet.

In einem vorteilhaften Verfahren werden die Messwerte einer dem Sensor nachgeordneten Signalauswertevorrichtung zugeführt, darin ausgewertet, und die ausgewerteten Daten zumindest teilweise wieder dem Sensor zugeführt und in dessen nichtflüchtigem Speicher abgelegt. Der Sensor kann auch zeitlich nacheinander an verschiedene Signalauswertevorrichtungen angeschlossen werden, welche jeweils die ausgewerteten Daten im Speicher des Sensors ablegen. Eine Signalauswertevorrichtung kann, falls erforderlich, auch auf die im Sensor gespeicherten Daten, beispielsweise die Patientendaten zugreifen.

Der erfindungsgemässe Sensor ist zum Beispiel auch zur längerfristigen Patientenüberwachung geeignet, insbesondere auch in Notfallsituationen, indem der Sensor beispielsweise am Ohr des Patienten befestigt wird, und der Sensor ständig am Patienten verleibt und die Daten der Signalauswertevorrichtungen, mit welchen dieser jeweils nacheinander verbunden wird, zumindest teilweise speichert. Somit ist es möglich mit einem einzigen Sensor einen Patienten kontinuierlich und im wesentlichen lückenlos zu überwachen, beginnend beispielsweise am Unfallort, und nachfolgend im Krankenwagen, im Operationssaal bis hin zur Aufwachstation. Dank dem, dass die gesamten Messdaten an jeder Station zur Verfügung stehen, und zudem ev. noch weitere Patientendaten zur Verfügung stehen, stehen auch in Notfallsftuationen jederzeit zuverlässige Daten zur Verfügung, was eine optimale Betreuung eines Patienten ermöglicht.

Die Erfindung wird nachfolgend an Hand von Ausführungsbeispielen beschrieben. Es zeigen:
- Fig. 1: einen Längsschnitt durch einen erfindungsgemässen Sensor;
- Fig. 2: eine Draufsicht auf einen Sensor;
- Fig. 3: schematisch ein Blockdiagramm des Sensors;
- Fig. 4: schematisch ein weiteres Blockdiagramm eines Sensors;
- Fig. 5: ein Detailaspekt des Sensorsignalprozessors;
- Fig. 6: mehrere mit einer Signalauswertevorrichtung verbundene Sensoren;
- Fig. 7: eine Korrekturkurve für eine Messvorrichtung;
- Fig. 8: einen Längsschnitt durch einen weiteren erfindungsgemässen Sensor, welcher an einem Ohrläppchen anliegt;
- Fig. 9: einen Längsschnitt durch einen weiteren erfindungsgemässen Sensor;
- Fig. 10: einen Querschnitt durch eine Mikro-pH-Elektrode;
- Fig. 11: einen Längsschnitt durch eine Atemgasmessvorrichtung;
- Fig. 12: einen Längsschnitt durch eine weitere Atemgasmessvorrichtung.

Im folgenden werden für dieselben Gegenstände dieselben Bezugszeichen verwendet.

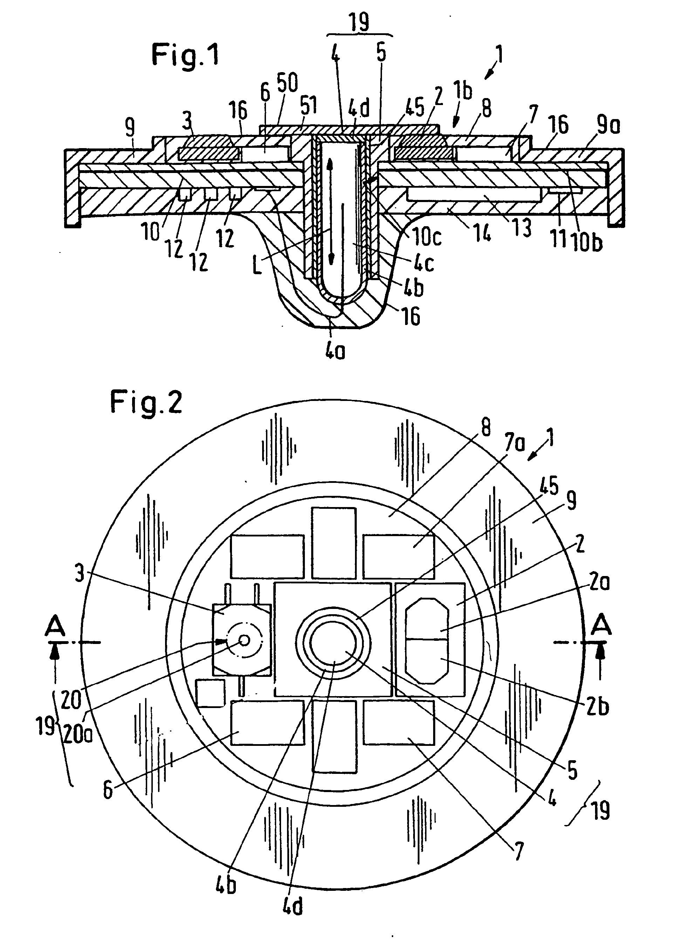

Der in Fig. 1 dargestellte Sensor 1 erlaubt eine kombinierte Messung der arteriellen Sauerstoffsättigung (SpO₂) und des transkutanen CO₂-Partialdrucks (tcpCO₂). Zur Messung der Sauerstoffsättigung weist der Sensor 1 ein Pulsoximetrie-Messystem 17 auf, welches eine zweifarbige Leuchtdiode 2 (LED) sowie einen Fotodetektor 3 umfasst. Die zweifarbige Leuchtdiode 2 umfasst zwei dicht nebeneinanderliegende, in einem gemeinsamen Gehäuse angeordnete Leuchtdioden 2a, 2b, wobei die eine Leuchtdiode 2a eine Wellenlänge von etwa 660 nm (Rot) und die andere Leuchtdiode 2b eine Wellenlänge von 890 nm (Infrarot) aufweist. Der Sensor 1 weist eine Oberfläche 1 b auf, über welcher im dargestellten Ausführungsbeispiel eine Membran 50 und dazwischen ein dünne Schicht Elektrolyt 51 angeordnet ist. Diese Membran 50 wird an einer gut durchbluteten Stelle des menschlichen Körpers auf die Haut aufgelegt, zum Beispiel an einem Finger oder am Ohrläppchen. Das von den beiden Leuchtdioden 2a,2b ausgestrahlte Licht durchstrahlt den sich über den. Leuchtdioden 2a,2b befindlichen Elektrolyt 51 sowie die Membran 50, und wird in das nicht dargestellte, gut durchblutete Körperteil geleitet und dort gestreut und teilweise absorbiert. Das vom Körperteil reflektierte Licht wird mit dem Fotodetektor 3 gemessen. Das vom Fotodetektor 3 gemessene Signal wird einem digitalen Sensorsignalprozessor 13, abgekürzt auch als DSSP bezeichnet, zugeleitet.

Der dargestellte Sensor 1 umfasst zudem eine elektrochemische Messvorrichtung 19 zur Messung des transkutanen Kohlendioxidpartialdrucks tcpCO₂, wobei diese Messvorrichtung 19 eine Mikro-pH-Elektrode 4 sowie eine Ag/AgCl-Referenzelektrode 5 umfasst. Der transkutane Kohlendioxidpartialdruck wird potentiometrisch gemessen, indem der pH-Wert der dünnen Schicht der Elektrolytlösung 51 gemessen wird, welche über die gut gasdurchlässige, hydrophobe Membran 50 mit der Haut in Verbindung steht. Eine Änderung des pCO₂-Wertes an der Hautoberfläche bewirkt eine pH-Änderung der Elektrolytlösung, die sich proportional zum Logarithmus der pCO₂-Änderung verhält. Der pH-Wert wird gemessen indem das Potential zwischen der Miniatur-pH-Elektrode 4 und der Ag/AgCl-Referenzelektrode 5 gemessen wird. Die Mikro-pH-Elektrode 4 ist über den elektrischen Innenableiter 4a Signal leitend mit dem digitalen Sensorsignalprozessor 13 verbunden.

Der dargestellte Sensor 1 umfasst zudem ein Erwärmungssystem 18 umfassend eine als elektrischer Widerstand ausgestaltete Erwärmungsvorrichtung 6 sowie einen Temperatursensor 7 zur Temperaturregelung. Das Erwärmungssystem 18 wird vorteilhafterweise in Kombination mit der elektrochemischen Messvorrichtung 19 verwendet, um über die Sensoroberfläche 1 b die sich darunter befindliche Haut zu erwärmen. Zur transkutanen Messung des Kohlendioxidpartialdrucks pCO₂ oder des Sauerstoffpartialdrucks pO₂ wird die Sensoroberfläche 1b beispielsweise auf eine Temperatur von etwa 40 °C bis 45 °C erwärmt.

Der Sensor 1 umfasst eine mehrschichtige, starre Leiterplatte 10, welche mit elektronischen Komponenten 2,3,6,7,12,13 bestückt ist, und welche eine Vielzahl nicht dargestellte elektrische Leiterbahnen aufweist, um die elektronischen Komponenten wie die Leuchtdiode 2, den Fotodetektor 3, den Widerstand 6, den Temperatursensor 7, einen zweiten Temperatursensor 7a oder weitere elektronische Komponenten wie Verstärker 12, 12a Signal leitend zu verbinden, insbesondere mit dem digitalen Sensorsignalprozessor 13. Alle elektronischen Komponenten sind in SMD-Technik (Surface Mounted Device) ausgestaltet, was den Vorteil ergibt, dass die Leiterplatte 10 automatisch bestückbar ist und somit sehr kostengünstig herstellbar ist. Im Zentrum der kreisförmigen Leiterplatte 10 ist eine als rundes Loch ausgestaltete Durchbrechung 10c ausgespart, in welcher die elektrochemische Messvorrichtung 19 umfassend die Mikro-pH-Elektrode 4 sowie die Ag/AgCl-Referenzelektrode 5 angeordnet ist. Die Mikro-pH-Elektrode 4 ist als Glaselektrode ausgestaltet, und weist einen Innenelektrolyt 4c auf, welcher von einem Schaftglas 4b sowie einem Membranglas 4d umgeben ist. Das im Innenelektrolyt 4c auftretende Potential wird über den Innenableiter 4a einem Verstärker 12 zugeführt. Zwischen der Mikro-pH-Elektrode 4 und der Referenzelektrode 5 ist ein Isolator 45 angeordnet.

Innerhalb der Leiterplatte 10 ist eine im wesentlichen vollflächige, elektrisch leitende Schicht 10b angeordnet, welche mit der elektrischen Masse verbunden ist, und welche zur Abschirmung elektrischer Felder dient. Etliche nicht dargestellte Leiterbahnen verlaufen isoliert und quer durch die elektrisch leitende Schicht 10b um die oberhalb und unterhalb auf der Leiterplatte 10 angeordneten elektronischen Komponenten zu verbinden. Mit Ausnahme der durch die quer verlaufenden Leiterbahnen bedingten Durchbrechungen ist die Schicht 10b vollflächig ausgestaltet.

Die wärmeempfindliche Elektrode 4 ist mit einem elektrisch leitenden Epoxyharz auf die Leiterplatte 10 geklebt, wobei dieser Klebstoff derart angeordnet ist, dass dieser zwischen der bei der Durchbrechung 10c mündenden Schicht 10b und der Ag/AgCl-Referenzelektrode 5 eine elektrisch leitende Verbindung bildet. Die Referenzelektrode 5 ist somit mit der Masse verbunden und bildet zudem eine Abschirmung gegen elektrische Felder.

Die Leiterplatte 10 ist in einem Gehäuse 9 gehalten. Das Gehäuse 9 besteht aus einem mit einer elektrisch leitenden Oberfläche wie einer Metallschicht versehenen Kunststoffkörper. Die oberhalb der Leiterplatte 10 angeordneten elektronischen Komponenten sind von einer wärmeleitenden Abdeckung 8 umgeben, welche vorzugsweise als wärmeleitender Verguss ausgestaltet ist. Vorzugsweise ist der Raum zwischen der Oberfläche der Leiterplatte 10 und der Sensoroberfläche 1 b mit einem wärmeleitenden, elektrisch isolierenden Epoxyharz vergossen. Die Sensoroberfläche 1 b ist derart bearbeitet, dass die mit ihrer Stirnfläche an die Oberfläche mündenden Komponenten Leuchtdiode 2, Fotodetektor 3 und elektrochemische Messvorrichtung 19 und der wärmeleitende Verguss 8 eine ebene Oberfläche bilden. Der wärmeleitende Verguss 8 weist den Vorteil auf, dass die vom Widerstand 6 erzeugte Wärme mit geringem Verlust und gleichmässig verteilt zur Sensoroberfläche 1b übertragbar ist, sodass die während dem Messen an der Membran 50 anliegende Haut gleichmässig erwärmbar ist.

Die unterhalb der Leiterplatte 10 angeordneten elektronischen Komponenten 12,13 sind von einer elektrisch isolierenden Abdeckung 14 umgeben. Für diese Abdeckung 14 wird vorzugsweise ein elektrisch hochisolierender Epoxyharz verwendet, welcher eingegossen wird. Der von der Elektrode 4 zur Leiterplatte 10 verlaufende Innenableiter 4a ist im hochisolierenden Epoxyharz eingebettet, um störende elektrische Einflüsse weitgehend zu unterdrücken.

Die Abdeckung 14, das Gehäuse 9 sowie Teile der wärmeleitenden Abdeckung 8 sind von einer metallischen Abdeckung 16 umgeben, um das Innere des Sensors 1 vor störenden elektrischen und elektromagnetischen Einflüssen zu schützen. Die innerhalb der Leiterplatte 10 oder an deren Oberfläche angeordnete, flächendeckende metallische Schicht 10b ist nicht unbedingt erforderlich, weist jedoch unter anderem den Vorteil auf, dass dadurch die Ausbreitung von innerhalb des Sensors 1 erzeugten, störenden elektromagnetischen Einflüssen verhindert wird, da die oberhalb der Leiterplatte 10 angeordneten elektronischen Komponenten 2,3,6,7 von den unterhalb der Leiterplatte angeordneten elektronischen Komponenten 12,13 abgeschirmt sind. Die elektronischen Komponenten 2,3,6,7,12,13 sind zudem derart im Sensor 1 verteilt angeordnet, dass sie möglichst keine gegenseitig störenden Einflüsse ausüben. Wie aus dem Schnitt gemäss Fig. 1 ersichtlich ist der hochohmige und daher störungsempfindliche Innenableiter 4a nach links verlaufend einem Verstärker 12 zugeführt, wogegen der digitale Sensorsignalprozessor 13 auf der rechten Seite angeordnet ist. Diese Komponenten sind somit örtlich getrennt, wobei sich zur zusätzlichen Abschirmung dazwischen noch die auf Masse liegende Referenzelektrode 5 befindet. Der digital arbeitende Sensorsignalprozessor 13 ist somit von den übrigen, analog arbeitenden elektronischen Komponenten 2,3,6,7,12 abgeschirmt. Der Sensor 1 mit derart angeordneten Komponenten und Abschirmungen ermöglicht eine Signalverarbeitung mit einem äusserst geringen Störsignalanteil.

Die Elektrode 4 ist länglich ausgestaltet und weist eine Längsrichtung L auf. Die Leiterplatte 10 ist senkrecht zur Längsrichtung L verlaufend und innerhalb der Länge der Elektrode 4 angeordnet. Diese Anordnung der Leiterplatte 10 bezüglich der Elektrode 4 weist den Vorteil auf, dass die Leuchtdiode 2, der Fotodetektor 3 und die Erwärmungsvorrichtung 6 nahe der Sensoroberfläche 1b zu liegen kommen, beziehungsweise dass die elektrischen Verbindungsleitungen sehr kurz sind. Diese Anordnung weist unter anderem den Vorteil eines geringen Störsignalanteils auf.

Der Sensor 1 umfasst zudem elektrische Anschlüsse 11, welche Signal leitend mit dem Sensorsignalprozessor 13 verbunden sind, und an welchen ein nicht dargestelltes Kabel anschliessbar ist, welches die elektrischen Signale einer nachgeordneten Signalauswertevorrichtung 37 zuführt. Die Figur 1 zeigt im wesentlichen einen Schnitt entlang der Linie A-A gemäss Fig. 2. Der in Figur 1 dargestellte Sensor 1 weist bevorzugt ein Gesamtgewicht von weniger als 10 Gramm auf. Der in Fig. 2 dargestellte Durchmesser des Sensors 1 ist in einer vorteilhaften Ausgestaltung derart gewählt, dass die Sensorauflagefläche das Ohrläppchen einer Person vollständig oder in wesentlichen Teilen abdeckt.

Eine mögliche Anordnung der in Figur 1 beschriebenen Komponenten ist in der in Figur 2 dargestellten Aufsicht auf die Sensoroberfläche 1b dargestellt. Zwischen der Mikro-pH-Elektrode 4 und der Referenzelektrode 5 ist ein Isolator 45 angeordnet.

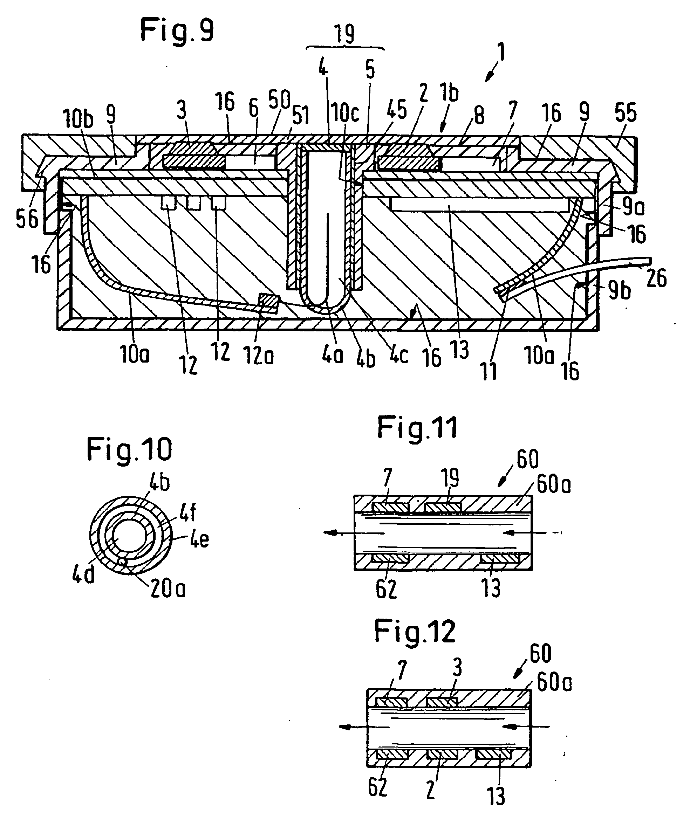

Im Sensor 1 könnte zudem eine weitere elektrochemische Messvorrichtung 19 zur Messung des transkutanen Sauerstoffpartialdrucks (tcpO₂) angeordnet sein. Wie in Figur 2 angedeutet, könnte der Fotodetektor 3 durch eine Elektrode 20 nach Clark ersetzt sein, welche einen in deren Zentrum angeordneten Platindraht 20a aufweist. Die Elektrode 20 bildet zusammen mit der Ag/AgCl-Referenzelektrode 5 die weitere elektrochemische Messvorrichtung 19 zur Messung des Sauerstoffpartialdrucks pO₂. Bei dieser Messmethode könnte auf die Leuchtdiode 2 verzichtet werden. Der Platindraht 20a könnte auch innerhalb des Fotodetektors 3 oder der Leuchtdiode 2 verlaufend angeordnet sein, wobei dessen Stirnfläche an die Oberfläche mündet. Der Platindraht 20a könnte auch, wie in dem in Fig. 10 dargestellten Schnitt durch die Mikro-pH-Elektrode 4 ersichtlich, zwischen einem äusseren Schaftglas 4e und einem inneren Schaftglas 4b angeordnet sein, wobei die Stirn des Platindrahtes 20a bis zur Oberfläche des Membranglases 4d mündet. Im Sensor 1 können Messvorrichtungen zur Messung unterschiedlichster physiologischer Parameter angeordnet sein. Beispielsweise könnte im Sensor 1 eine an sich bekannte Messvorrichtung zum Messen der Kerntemperatur Tk des menschlichen Körpers angeordnet sein, welche Mittels Infrarot, d.h. mit einem Infrarotsender und -empfänger, mit Hilfe einer Messung am Trommelfell des Ohr die Kerntemperatur Tk zu ermitteln erlaubt. Im Sensor 1 könnten auch weitere, an sich bekannte Messvorrichtungen angeordnet sein, beispielsweise zur Messung des Hematokrit (HCT) oder des Blutdrucks (CNIBP). Erfindungsgemäss sind im Sensor 1 mehrere derartiger Messvorrichtungen angeordnet

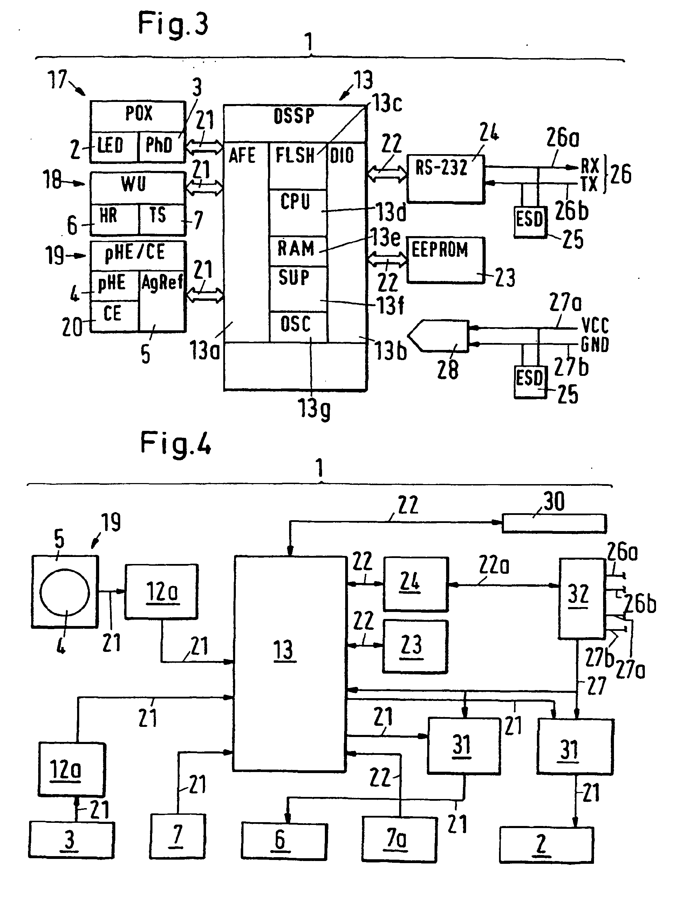

Fig. 3 zeigt mit einem Blockdiagramm schematisch die im Sensor 1 angeordneten elektronischen Komponenten. Von besonderer Bedeutung ist der im Sensor 1 angeordnete digitale Sensorsignalprozessor 13, welcher im Sensor 1 eine weitgehend digitale Signalverarbeitung ermöglicht. Ein derartiger Sensorsignalprozessor 13, im Englischen auch als "Digital Sensor Signal Processor" (DSSP) bezeichnet, umfasst üblicherweise:
- zumindest einen analogen Ein- und Ausgang 13a (AFE) mit zumindest einem Analog-Digital-Wandler 35 sowie zumindest einem Digital-Analog-Wandler 36,
- einen digitalen Ein- und Ausgang 13b (DIO),
- einen nichtflüchtigen Speicher 13c (FLSH),
- einen Zentralprozessor 13d (CPU),
- einen Arbeitsspeicher 13e (RAM).

Der Zentralprozessor 13d steuert die Signalverarbeitung im Sensors 1. Die Software zur Ansteuerung des Zentralprozessors 13d ist im nichtflüchtigen Speicher 13c abgelegt. Diese Software bestimmt wie die einzelnen Komponenten des Sensorsignalprozessors 13 und die Messvorrichtungen 17,18,19 angesteuert werden, und wie die digitalen Daten mit einer übergeordneten Signalauswertevorrichtung 37 ausgetauscht werden.

Der Sensorsignalprozessor 13 kann weitere Komponenten umfassen wie beispielsweise ein Oszillator 13g (OSC) oder eine Statusüberwachungsvorrichtung 13f (SUP), welche den Sensorsignalprozessor 13 beispielsweise bei einem Spannungsausfall in einen definierten Ausgangszustand zurücksetzt.

Der Sensorsignalprozessor 13 ist über analoge Signalleitungen 21 mit zumindest einer der Messvorrichtungen 17, 18, 19 verbunden. Die optische Messvorrichtung 17 umfasst die Leuchtdiode 2 und den Fotodetektor 3. Das Erwärmungssystem 18, umfasst den elektrischen Widerstand 6 und den Temperatursensor 7. Die elektrochemische Messvorrichtung 19 umfasst die Referenzelektrode 5 und die Mikro-pH-Elektrode 4 zur tcpCO₂-Messung nach Stow-Severinghaus und/oder die Elektrode 20 zur tcpO₂-Messung nach Clark. Vorzugsweise umfasst der Sensor 1 zwei Temperatursensoren, einen digitalen Temperatursensor 7 sowie einen analogen Temperatursensor 7a. Im Vergleich zu einem rein analog arbeitenden Sensor weist der erfindungsgemässe, im wesentlichen digitale Sensor 1 eine wesentlich höhere Redundanz auf. Der Zustand des Sensors ist zudem wesentlich differenzierter überwachbar. So kann beispielsweise festgestellt werden, ob ein digitales Signal anliegt oder nicht. Über den gesamten vom Sensor 1 verbrauchten Strom kann auf dessen Erwärmung geschlossen werden, und falls erforderlich, die Stromzufuhr bzw. die Heizleistung reduziert werden. Durch die Verwendung von zwei Temperatursensoren 7 kann deren Temperatur regelmässig verglichen werden, und bei einer grösseren Abweichung darauf geschlossen werden, dass einer der Temperatursensoren 7 defekt ist. Der erfindungsgemässe Sensor 1 weist somit den Vorteil auf, dass dieser wesentlich zuverlässiger arbeitet, dass mögliche Störungen frühzeitig erkennbar sind, und dass zu hohe Temperaturen im Sensor 1, welche beispielsweise Gewebe wie das Ohrläppchen schädigen könnten, ausgeschlossen sind.

Der Sensorsignalprozessor 13 ist über den digitalen Ein- und Ausgang 13b und über eine digitale Signalleitung 22 mit einer seriellen Schnittstelle 24, auch als UART bezeichnet (Universal Asynchronous Receiver Transmitter), verbunden, welche über zwei serielle Leiter 26a, 26b einen bidirektionalen Datenaustausch über ein Verbindungsmittel 26 zu der übergeordneten Signalauswertevorrichtung 37 ermöglicht. Im dargestellten Ausführungsbeispiel ist die serielle Schnittstelle 24 entsprechend dem Standard RS-232 ausgestaltet. Eine elektrostatische Ableitvorrichtung 25 (ESD) schützt die serielle Schnittstelle 24 vor Überspannungen.

Der Sensorsignalprozessor 13 kann zudem über die digitale Signalleitung 22 mit einem Zusatzspeicher 23 verbunden sein, wobei der Zusatzspeicher 23 vorzugsweise als EEPROM ausgestaltet ist, was in Englisch als "Electrical Eraseable Programmalbe Read Only Memory" bezeichnet wird. Die einzelnen Komponenten des Sensors 1 werden über eine Energieversorgung 28 mit elektrischem Strom versorgt. Der Energieversorgung 28 wird über zwei Leiter eine Speisespannung 27a (VCC) sowie eine Masse 27b (GND) zugeführt.

Es könnte auch nur ein zweipoliges Kabel umfassend die beiden Leiter 27a,27b verwendet werden, indem die digitalen Signale entsprechend auf diese Leiter 27a, 27b aufmoduliert werden, sodass der bidirektionale Datenaustausch ebenfalls über die Leiter 27a, 27b erfolgt, wodurch auf die beiden Leiter 26a, 26b verzichtet werden kann.

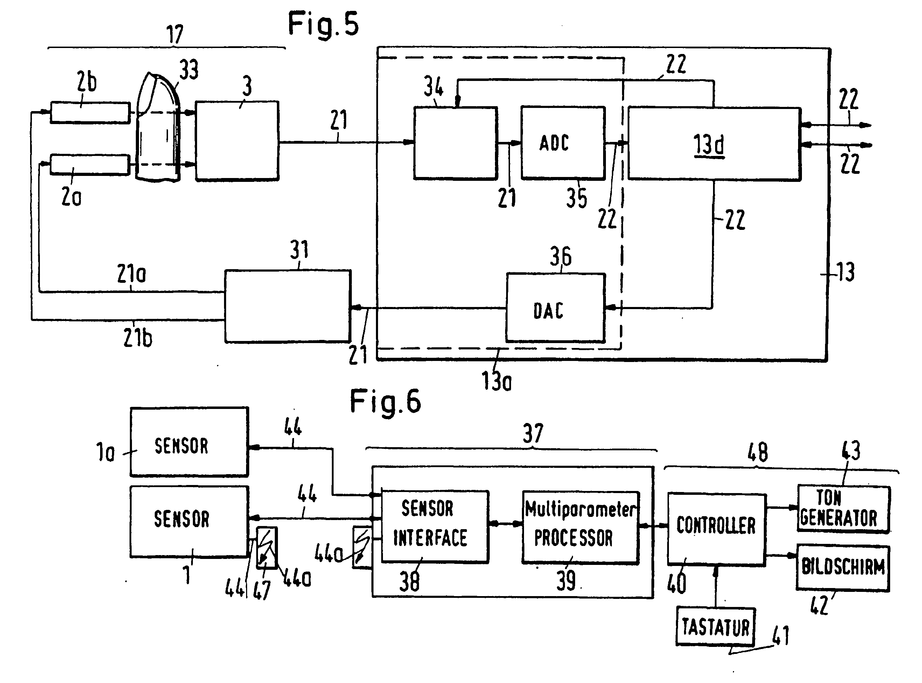

Das in Fig. 5 dargestellte Blockschaltbild zeigt am Beispiel einer optischen Messvorrichtung 17 ein Detailaspekt des im Sensor 1 angeordneten, digitalen Sensorsignalprozessors 13. Der analoge Ein- und Ausgang 13a umfasst einen analogen, ansteuerbaren Signalverstärker 34, welchem über eine analoge Signalleitung 21 ein Analog-Digital-Wandler 35 mit 16 Bit Auflösung nachgeschaltet ist. Das derart erzeugte digitale Signal wird über eine digitale Signalleitung 22 dem Zentralprozessor 13d zugeführt. Der Fotodetektor 3 ist über die analoge Signalleitung 21 mit dem Signalverstärker 34 verbunden. Der Verstärkungsfaktor des Signalverstärkers 34 kann vom Zentralprozessor 13d über die digitale Signalleitung 22 angesteuert werden. Um das Auflösevermögen des Analog-Digital-Wandlers 35 optimal zu nutzen wird ein schwaches Signal des Fotodetektors 3 nach einer per Software dem Zentralprozessor 13d vorgegebenen Regel verstärkt.

Die in Figur 5 dargestellte optische Messvorrichtung 17 zur pulsoximetrischen Messung der Sauerstoffsättigung (SpO₂) misst das durch den Finger 33 transmittierte Licht, in dem auf der einen Seite des Fingers 33 die beiden Leuchtdioden 2a, 2b angeordnet sind, und auf der anderen Seite der Fotodetektor 3. Die Leuchtdioden 2a,2b sind über analoge Signalleitungen 21 a, 21 b mit einer Umschaltvorrichtung 31 verbunden, welche jeweils eine der Leuchtdioden 2a, 2b mit Spannung versorgt. Der Zentralprozessor 13d ist über die digitale Signalleitung 22 mit dem Digital-Analog-Wandler 36 (DAC) verbunden, welcher ein analoges Signal 21 zur Ansteuerung der Umschaltvorrichtung 31 generiert. Wesentliche Komponenten des Sensorsignalprozessors 13 sind somit der Analog-Digital-Wandler 35 sowie der Digital-Analog-Wandler 36, dank welchen die analog arbeitende Messvorrichtung 17 betreibbar ist. Ein wesentlicher Vorteil des digitalen Sensorsignalprozessors 13 ist darin zu sehen, dass die Messvorrichtung 17 durch den Zentralprozessor 13d beziehungsweise durch dessen Software angesteuert wird. An Stelle der in Fig. 5 beispielhaft dargestellten optischen Messvorrichtung 17 kann eine Vielzahl weiterer Messvorrichtungen oder Regelsysteme, beispielsweise die elektrochemische Messvorrichtung 19 oder das Erwärmungssystem 18, wie dargestellt mit dem digitalen Sensorsignalprozessor 13 verbunden werden.

Es erweist sich als besonders vorteilhaft den Sensorsignalprozessor 13 in Kombination mit der optischen Messvorrichtung 17 zur pulsoximetrischen Messung zu verwenden. Es ist bekannt, dass die Empfindlichkeit der pulsoximetrischen Messung dadurch begrenzt ist, dass die Messsignale durch Störsignale und elektronisches Rauschen überlagert sind. Der erfindungsgemässe Sensor 1 wandelt das analoge Signal des Fotodetektors 3 mit Hilfe des Sensorsignalprozessors 13 innerhalb des Sensors 1 in ein digitales Signal um. Die analoge Signalleitung 21 ist sehr kurz und zudem gegen aussen durch die metallische Abdeckung 16 abgeschirmt, sodass das analoge Signal des Fotodetektors 3 kaum durch Störsignale oder elektronisches Rauschen beeinträchtigt wird. Das digitale Signal kann über die digitale Signalleitung 22 ohne Qualitätsverlust der übergeordneten Signalauswertevorrichtung 37 zugeführt werden. Es ist bekannt, dass die primären, das heisst, die vom Fotodetektor 3 gemessenen Messsignale, bei der pulsoximetrischen Messung sehr schwach sein können. Der erfindungsgemässe Sensor 1 ermöglicht Störsignale und elektronisches Rauschen weitgehend zu vermeiden, sodass auch Messungen mit schwachen primären Messsignalen noch ausgewertet werden können.

Die in Fig. 5 dargestellte Anordnung wird beispielsweise vom Zentralprozessor 13d derart angesteuert, dass der Fotodetektor 3 nacheinander das Licht der infraroten Leuchtdiode 2a, das Licht der roten Leuchtdiode 2b, und danach, indem keine der Leuchtdioden 2a,2b angesteuert wird, das durch die Umgebung bewirkte Licht misst. Diese drei Messwerte werden beispielsweise innerhalb von 16 Millisekunden 4 mal erfasst, und danach als Datenpaket, umfassend 12 Werte mit je 16 Bit, über die digitale Signalleitung 22 der übergeordneten Signalauswertevorrichtung 37 übermittelt. In diesem Datenpaket können auch weitere Werte, wie die Temperatur oder die Messwerte der elektrochemischen Messvorrichtung 19 übermittelt werden.

In einer weiteren vorteilhaften Ausgestaltung werden die von der Messvorrichtung 17, 18, 19 gemessenen Signale durch den Sensorsignalprozessor 13 in ein normiertes digitales Ausgangssignal gewandelt. Beispielsweise wird die im Sensor 1 mit dem Temperatursensor 7 gemessene Temperatur derart normiert, dass der digitale Wert 0 dem Wert 0°C entspricht, und der digitale Wert 10000 dem Wert 100°C entspricht.

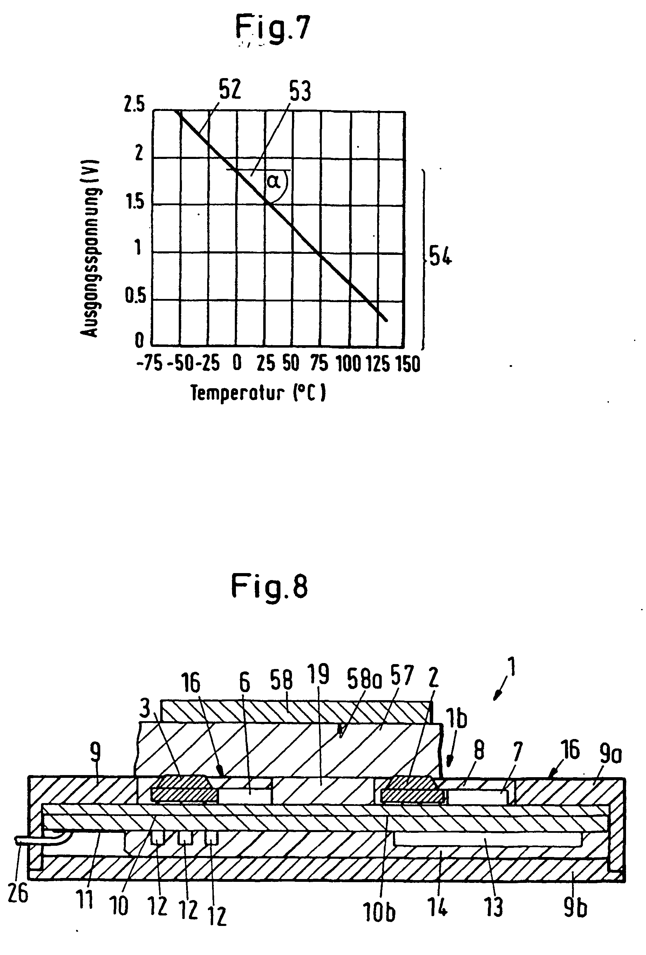

Um die Genauigkeit der gemessenen Werte weiter zu verbessern sind im Sensor 1 in einer weiteren vorteilhaften Ausgestaltung die Kennwerte zumindest eines der im Sensor 1 verwendeten elektronischen Bauteile gespeichert, beispielsweise die Kennlinie des Fotodetektors 3, der Leuchtdioden 2a, 2b, oder wie in Fig. 7 dargestellt, die Kennlinie 52 des Temperatursensors 7. Die Werte der Kennlinie 52 werden vorzugsweise im Zusatzspeicher 23 gespeichert. Die Kennlinie 52 kann auf unterschiedlichste Weise gespeichert werden, beispielsweise als Polygonzug. Die in Fig. 7 dargestellte Kennlinie 52 ist im wesentlichen eine Gerade, sodass die Charakteristik der Kennlinie 52 durch das Abspeichern der Steigung 53 sowie des Offsets 54 eindeutig bestimmt ist. Derart können die Kennlinien 52 von einigen oder allen wesentlichen elektronischen Komponenten des Sensors 1 im Zusatzspeicher 23 gespeichert werden.

Das Abspeichern der Kennlinien 52 ergibt die folgenden, entscheidenden Vorteile:
- Auf Grund der Fertigungstoleranzen weist jede elektronische Komponente eine individuelle Streuung auf, weshalb sich für jede elektronische Komponente eine individuelle Kennlinie 52 ergibt. Weil diese individuelle Kennlinie 52 im Sensor 1 gespeichert ist kann der Zentralprozessor 13d auf diese Werte zugreifen und die gemessenen analogen Signale äusserst genau in normierte digitale Werte umrechnen. An Stelle dieser individuellen Kennlinie 52 kann jedoch auch nur eine Kennlinie 52 gespeichert werden, welche beispielsweise für eine Serie von Bauteilen dieselben Werte aufweist.
- Die Messgenauigkeit des Sensors 1 wird erhöht.
- Der Sensor 1 muss nur in relativ grossen Zeitabständen kalibriert werden. Zudem wird das Kalibrieren vereinfacht. Beispielsweise weist die Elektrode 4 eine Kennlinie mit einer konstanten, vom Elektrolyen 51 unabhängigen Steigung auf. Da der Elektrolyt 51 mit der Zeit austrocknet verändert sich jedoch der Offset dieser Kennlinie, sodass beim Kalibrieren nur der Offset neu zu bestimmen ist, wogegen der Wert der Steigung nicht neu eingegeben werden muss.
- Eine Alterung der elektronischen Komponenten, beispielsweise der Leuchtkraft der Leuchtdioden, kann vom digitalen Sensorsignalprozessor 13 mit Hilfe des Fotodetektors 3 selbsttätig erfasst werden, und die alterungsbedingt veränderte Kennlinie 52 anstelle der ursprünglichen Kennlinie 52 im Zusatzspeicher 23 abgelegt werden.
- Die digitalen Ausgangswerte jedes Sensors 1 sind einheitlich vorgegeben. Somit kann beispielsweise ein defekter Sensor 1 problemlos ersetzt werden. Eine Kalibrierung des Sensors, beziehungsweise eine Anpassung an die übergeordnete Auswertevorrichtung 37, ist nicht erforderlich.

Im Zusatzspeicher 23 können weitere Daten gespeichert werden, beispielsweise eine individuelle Nummer für jeden Sensor 1, oder eine Bezeichnung für den Typ des Sensors 1, sodass die übergeordnete Auswertevorrichtung 37 selbsttätig die Eigenschaften des Sensors 1 erkennen kann. Im Zusatzspeicher 23 können auch Patientendaten gespeichert werden, sodass diese beim Umstecken eines Sensors 1 der einer neuen Auswertevorrichtung 37 sofort zur Verfügung stehen. Im Zusatzspeicher 23 könnten auch von der Auswertevorrichtung 37 ausgewertete Daten gespeichert werden, sodass im Sensor 1 zumindest ein Teil der ausgewerteten Daten gespeichert werden. Beim Umstecken des Sensors 1 an eine weitere Auswertevorrichtung 37 stehen dieser die gesamten im Sensor 1 gespeicherten Daten zur Verfügung.

Der Sensorsignalprozessor 13 kann auch einen Betriebsstundenzähler umfassen, mit welchem die gesamte Betriebsdauer des Sensors 1 erfasst wird. Falls das Alterungsverhalten eines elektronischen Bauteils, beispielsweise der LED 2, bekannt ist, kann die zu erwartende Veränderung der Kennlinie korrigiert werden.

In einem bevorzugten Verfahren wird nur die Mikro-pH-Elektrode 4 oder die Elektrode 20 periodisch neu kalibriert. Da der Elektrolyt 51 mit der Zeit Flüssigkeit verliert ist die Mikro-pH-Elektrode 4 immer wieder neu zu kalibrieren. Die zum Beispiel mit Hilfe eines Kalibrators jeweils neu ermittelten Werte können vom Sensorsignalprozessor 13 im Zusatzspeicher 23 abgelegt werden.

Fig. 6 zeigt schematisch ein Blockschaltbild eines Systems zum Messen physiologischer Parameter. Der Sensor 1 ist über das Kabel 44 signalleitend mit der übergeordneten Signalauswertevorrichtung 37 verbunden. Das Kabel 44 des Sensors könnte auch mit einem Sender/Empfänger 44a verbunden sein, um die digitale Information über eine kabellose Verbindung, beispielsweise Mittels elektromagnetischer Wellen, auf den zweiten Sender/Empfänger 44a zu übertragen. Der eine Sender/Empfänger 44a kann zusammen mit einer Batterie in einem Gehäuse 47 angeordnet sein. Der Sensor 1 wird durch diese Batterie mit elektrischer Energie versorgt. Die Signalauswertevorrichtung 37 umfasst im wesentlichen ein digitales Sensorinterface 38 und nachfolgend einen Rechner 39, auch als Multiparameter-Processor bezeichnet. Der Rechner 39 verwendet die dem jeweiligen Sensor 1 entsprechende Software, um die digitalen Daten auszuwerten. Weist der Sensor 1 beispielsweise die in Fig. 5 dargestellte optische Messvorrichtung 17 auf, so berechnet der Rechner 39 aus den gemessenen Werten mit Hilfe an sich bekannter Algorithmen die Sauerstoffsättigung SpO₂, und zusätzlich, falls erforderlich, die Pulsfrequenz. Dem Rechner 39 ist eine Ein- und Ausgabevorrichtung 48 nachgeordnet, mit einem Controller 40, welcher den Rechner 39, eine Tastatur 41, einen Bildschirm 42 sowie einen Tongenerator 43 ansteuert. Die Signalauswertevorrichtung 37 könnte auch innerhalb des Sensors 1 angeordnet sein. Die Signalauswertung könnte auch bereits im Sensorsignalprozessor erfolgen, indem dieser mit einer entsprechenden Software betrieben wird, sodass am Ausgang des Sensors 1 bereit das ausgewertete Messsignal, beispielsweise die Sauerstoffsättigung des Hämoglobins im arteriellen Blut und/oder der transkutane Kohlendioxidpartialdruck (tcpCo2) und/oder die Pulsfrequenz zur Verfügung stehen.

Zur Realisierung der Signalauswertevorrichtung 37 sowie der Ein- und Ausgabevorrichtung 48 kann in einer vorteilhaften Ausgestaltung ein handelsüblicher Computer, insbesondere auch ein als "Palmtop" bezeichnetes Handgerät verwendet werden, welches sehr leicht und kostengünstig ist, und zudem eine Visualisierung der gemessenen Werte erlaubt. In einer einfachen Ausführungsform ist somit zur Messung beispielsweise der Sauerstoffsättigung sowie des Kohlendioxidpartialsdrucks nur ein Sensor erforderlich, welcher mit einem handelsüblichen "Palmtop" verbunden ist. Der Sensor liefert vorzugsweise die bereits ausgewerteten Messwerte, welche vom "Palmtop" im wesentlichen nur noch auf dessen Anzeigevorrichtung anzuzeigen sind.

Die Anordnung gemäss Fig. 6 weist den Vorteil auf, dass eine einzige Signalauswertevorrichtung 37 genügt um die Messsignale unterschiedlicher Sensoren 1 auszuwerten. Der Signalauswertevorrichtung 37 müssen unterschiedliche Softwareprogramme zur Verfügung stehen, sodass diese je nach Sensor 1 auf die entsprechende Auswertesoftware zugreifen kann. Die Hardware der Signalauswertevorrichtung 37 bleibt jedoch identisch. Da die Signalauswertevorrichtung 37 in einer bevorzugten Ausführungsform den Typ des Sensors 1 automatisch erkennt, können auch unterschiedliche Sensoren 1 problemlos mit dem Sensorinterface 38 verbunden werden, ohne dass zusätzliche Einstellungen erforderlich sind. In einer weiteren Ausführungsform können auch zwei oder noch mehr Sensoren 1, 1a mit derselben Signalauswertevorrichtung 37 verbunden werden.

Fig. 4 zeigt mit einem Blockschaltbild ein weiteres Ausführungsbeispiel eines Sensors 1. Die analogen Messwerte der elektrochemischen Messvorrichtung 19 oder des Fotodetektors 3 werden in einem lmpedanzwandler 12a verstärkt und danach über die analoge Signalleitung 21 dem digitalen Sensorsignalprozessor 13 zugeführt. Diesem wird zudem das Messsignal des Temperatursensors 7 zugeführt. Der Sensorsignalprozessor 13 ist über digitale Signalleitungen 22 mit dem Zusatzspeicher 23, dem digitalen Temperatursensor 7a, der seriellen RS-232-Schnittstelle 24 sowie, falls erforderlich, mit einer Programmiervorrichtung 30 verbunden. Die serielle Schnittstelle 24 ist über die serielle digitale Signalleitung 22a mit einer Kabelanschlussvorrichtung 32 verbunden, von welcher aus die Leiter 26a,26b,27a,27b in Form eines gemeinsamen Kabels der Signalauswertevorrichtung 37 zugeführt werden. Die Speisespannung 27a und die Masse 27b werden als Leitungen 27 den Verstärkern 31 sowie dem Sensorsignalprozessor 13 zugeführt. Der Sensorsignalprozessor 13 steuert über die analogen Signalleitungen 21 die Verstärker 31 und damit die zweifarbige Leuchtdiode 2 sowie den elektrischen Widerstand 6 an.

Im dargestellten Ausführungsbeispiel umfasst das Kabel vier Leiter 26a,26b, 27a, 27b. Dieses Kabel kann sehr dünn und flexibel ausgestaltet sein, was den Vorteil ergibt, dass sich eine Bewegung des Kabels nicht oder kaum auf die Lage des Sensors 1 auswirkt. Über einen einzigen Leiter 26a können auch bidirektional digitale Daten ausgetauscht werden, sodass zum Betrieb des Sensors 1 ein Kabel umfassend die Leiter 26a, 27a und 27b genügen würde. Ein derartiges Kabel kann besonderes dünn und flexibel ausgestaltet sein.

Fig. 8 zeigt in einem Längsschnitt ein weiteres Ausführungsbeispiel eines Sensors 1, welcher mit Hilfe einer Klammer 58 mit Licht reflektierender Oberfläche 58a an ein Ohrläppchen 57 geklammert ist. Im Unterschied zu dem in Fig. 1 dargestellten Ausführungsbeispiel ist die elektrochemische Messvorrichtung 19 als Halbleiter ausgestaltet, welcher das Kohlendioxid direkt und ohne einen Elektrolyten 51 zu messen erlaubt. Die Messvorrichtung 19 könnte auch als Festkörperelektrode ohne flüssigen Innenelektrolyt ausgestaltet sein. Die Messvorrichtung 19 kann auch als Halbleiter ausgestaltet sein, beispielsweise in Dickfilmtechnologie mit einer pH-sensitiven Schicht. Der in Fig. 8 dargestellte Sensor 1 weist ein Gehäuse 9 umfassend ein erstes und zweites Teilgehäuse 9a, 9b auf. Die gemessenen Signale werden über ein als Kabel ausgestaltetes Verbindungsmittel 26 abgeleitet. Fig. 9 zeigt in einem Längsschnitt ein weiteres Ausführungsbeispiel eines Sensors 1. Im Unterschied zu dem in Fig. 1 dargestellten Ausführungsbeispiel umfasst die Leiterplatte 10 zwei flexible Abschnitte 10a, welche mit Leiterbahnen versehen ist. Am Ende des einen flexiblen Abschnittes 10a ist ein Vorverstärker 12a angeordnet, welcher mit dem Innenableiter 4a verbunden ist. Diese Anordnung erlaubt das hochohmige und daher störungsempfindliche Signal der Innenableiters 4a nahe an der Austrittsstelle mit dem Vorverstärker 12a zu verstärken. Diese ermöglicht eine störungsfreie Signal leitende Verbindung zwischen der Elektrode 4 und der Leiterplatte 10. Der flexible Abschnitt 10a ermöglicht zudem eine kostengünstige Fertigung des Sensors, indem die Leiterplatte 10 mit den elektronischen Komponenten 2,3,6,7,12,13 bestückt wird, daraufhin die elektrochemische Messvorrichtung 19 auf die Leiterplatte 10 geklebt wird, daraufhin der Vorverstärker 12a mit dem Innenableiter 4a kontaktiert wird, und daraufhin der vom zweiten Gehäuseteil 9b und der Leiterplatte 10 begrenzte Raum mit einem elektrisch hochisolierenden Epoxyharz vergossen wird. Am anderen flexiblen Abschnitt 10a ist eine Kontaktstelle 11 sowie ein nach aussen führendes Kabel 26 angeordnet. In Fig. 9 ist zudem der in Fig. 1 nur andeutungsweise dargestellte Spannring 55 mit Membran 50 dargestellt, welcher über eine Schnappvorrichtung 56 mit dem Sensor 1 lösbar verbunden ist.

Figur 11 zeigt einen Längsschnitt durch einen als Atemgasmessvorrichtung 60 ausgestalteten Sensor 1. Im Innern des rohrförmigen Gehäuses 60a sind, nebst weiteren nicht dargestellten Komponenten, eine Messvorrichtung 19, ein Temperatursensor 7, ein Feuchtigkeitssensor 62 sowie ein digitaler Sensorsignalprozessor 13 angeordnet. In der in Figur 12 dargestellten Ausführungsform besteht die Messvorrichtung 19 aus den beiden Komponenten Leuchtdiode 2 und Fotodetektor 3. Diese Messvorrichtung 19 könnte auch als Halbleiterchip ausgestaltet sein, welcher Licht unterschiedlicher Wellenlängen zu erzeugen und/oder zu messen erlaubt, um ein Spektrum zu messen und unterschiedliche Gasanteile im Atemgas zu bestimmen.

In einer bevorzugten Ausgestaltung wird im Sensor 1 ein Sensorsignalprozessor mit sehr geringem Energiebedarf verwendet, was als ein Low-Power-Prozessor oder ein Ultra-Low-Power-Prozessor bezeichnet wird. Bevorzugt sind auch die übrigen elektronischen Bauteile im Sensor 1 für geringen Energiebedarf ausgelegt. Dadurch wird in Sensor 1 nur einen geringe Störungs- und Abstrahileistung erzeugt. Dies ergibt zudem den Vorteil, dass der Sensor 1 keine oder nur sehr geringe elektromagnetischen Strahlungen auf einen Menschen ausübt. Zudem genügt zur Energieversorgung ein sehr dünnes Kabel.

## Patentansprüche

1. Sensor (1) zum kombinierten Messen von zumindest zwei physiologischen Parametern wie Sauerstoff oder Kohlendioxid im Blut, umfassend Messvorrichtungen (17,19), umfassend ein Erwärmungssystem (18) mit einer Erwärmungsvorrichtung (6) sowie einem Temperatursensor (7), sowie umfassend ein elektrisch leitendes Verbindungskabel zu einer nachfolgenden Vorrichtung (37, 44a) wobei, im Sensor (1) ein digitaler Sensorsignalprozessor (13) angeordnet ist, welcher Signal leitend mit den Messvorrichtungen (17,19) sowie dem Erwärmungssystem (18) verbunden ist, der Sensorsignalprozessor (13) die Temperatur des Sensors (1) regelt, der Sensorsignalprozessor (13) ein digitales Ausgangssignal bereitstellt, der Sensor (1) Mittel (24) für einen bidirektionalen, digitalen Datenaustausch aufweist, und wobei das Verbindungskabel ein signalübertragendes, als zumindest zweipoliges Kabel (26a, 26b) ausgestaltetes Verbindungsmittel (26) aufweist, über welches die Übermittlung der digitalen Ausgangssignale mittels bidirektionalem Datenaustausch zur nachfolgenden Vorrichtung (37, 44a) erfolgt.

2. Sensor (1) nach Anspruch 1, **dadurch gekennzeichnet, dass** die Erwärmungsvorrichtung (6) als elektrischer Widerstand ausgestaltet ist und der elektrische Widerstand (6) von einem wärmeleitenden Verguss (8) umgeben ist.

3. Sensor (1) nach Anspruch 2, **dadurch gekennzeichnet, dass** der wärmeleitende Verguss (8) aus elektrisch isolierendem Epoxyharz besteht.

4. Sensor (1) nach Anspruch 2 oder 3, **dadurch gekennzeichnet, dass** der Sensor (1) eine zur Auflage am menschlichen Körper bestimmte Sensoroberfläche (1b) aufweist, und dass der wärmeleitende Verguss (8) ein Teil der Sensoroberfläche (1b) bildet.

5. Sensor (1) nach einem der vorhergehenden Ansprüche, **dadurch gekennzeichnet, dass** der Sensor (1) ein Gewicht von weniger als 10 Gramm, und vorzugsweise ein Gewicht von weniger als 5 Gramm aufweist.

6. Sensor (1) nach einem der vorhergehenden Ansprüche, umfassend einen Datenspeicher (23), in welchem eine Kennlinie zumindest einer Messvorrichtung (17, 19) speicherbar ist.

7. Sensor (1) nach einem der vorhergehenden Ansprüche zum kombinierten Messen der physiologischen Parameter Sauerstoff und Kohlendioxid im Blut, umfassend eine Messvorrichtung (17) zur Messung der arteriellen Sauerstoffsättigung sowie eine Messvorrichtung (19) zur Messung des arteriellen Kohlendioxid-Partialdrucks .

8. Sensor (1) nach einem der vorhergehenden Ansprüche, umfassend eine Mess- oder Auswertevorrichtung zum Erfassen der Pulsfrequenz.

9. Sensor (1) nach einem der vorhergehenden Ansprüche, **dadurch gekennzeichnet, dass** der Sensor (1) eine zur Auflage an einem Ohrläppchen bestimmte Sensorauflagefläche (1 b) aufweist.

10. Sensor (1) nach einem der vorhergehenden Ansprüche, umfassend eine Messvorrichtung (17, 19) aus folgender Gruppe:
- eine Messvorrichtung (19) zur Messung des CO₂-Gehaltes, insbesondere nach Stow-Severinghaus,
- eine Messvorrichtung (19) zur Messung des O₂-Gehaltes, insbesondere nach Clark,
- eine optische Messvorrichtung (17) mit Mitteln zur pulsoximetrischen Messung der arteriellen Sauerstoffsättigung umfassend eine LED (2;2a,2b) und einen Fotodetektor (3),
- eine Messvorrichtung zur Messung der Pulsfrequenz,
- eine Messvorrichtung zur Messung des Hematokrit,
- eine Messvorrichtung zur Messung des Blutdrucks ,
- eine Messvorrichtung zur Messung von Bestandteilen des Atemgases,
- eine Messvorrichtung zur Messung der Körpertemperatur,
- eine Messvorrichtung zur Messung der Feuchtigkeit.

11. Sensor (1) nach einem der vorhergehenden Ansprüche, **dadurch gekennzeichnet, dass** eine der Messvorrichtungen (17, 19) als elektrochemische Messvorrichtung ausgebildet ist, insbesondere zur Messung des transkutanen CO₂-Partialdrucks oder des transkutanen O₂-Partialdrucks.

12. Sensor (1) nach einem der vorhergehenden Ansprüche, **dadurch gekennzeichnet, dass** eine der Messvorrichtungen (17, 19) als Halbleiterchip (19) ausgebildet ist, und dass der Halbleiterchip (19) die Konzentration eines Gases wie CO₂, O₂, N oder eines Gasgemisches wie ein als Enflurane bezeichnetes Narkosegas zu messen erlaubt.

13. Sensor (1) nach einem der Ansprüche 1 bis 11, **dadurch gekennzeichnet, dass** eine der Messvorrichtungen (17, 19) als optische Messvorrichtung umfassend eine Lichtquelle (2) und einen Fotodetektor (3) ausgestaltet ist.

14. Sensor (1) nach Anspruch 13, **dadurch gekennzeichnet, dass** eine der Messvorrichtungen (17, 19) einen Halbleiterchip (19) umfasst, welcher Licht unterschiedlicher Wellenlänge zu erzeugen und/oder zu messen erlaubt.

15. Sensor (1) nach einem der vorhergehenden Ansprüche, umfassend einen Datenspeicher (23), in welchem gemessene Werte und/oder Patientendaten speicherbar sind.

16. Sensor (1) nach einem der vorhergehenden Ansprüche, umfassend eine zumindest teilweise starre Leiterplatte (10), welche mit elektronischen Komponenten (12,13) bestückt ist.

17. Sensor (1) nach Anspruch 16, **dadurch gekennzeichnet, dass** die Leiterplatte (10) mit einer Elektrode (4;20) bestückt ist.

18. Sensor (1) nach Anspruch 16 oder 17, **dadurch gekennzeichnet, dass** die Leiterplatte (10) einen flexiblen Abschnitt (10a) umfasst.

19. Sensor (1) nach Anspruch 18, **dadurch gekennzeichnet, dass** im flexiblen Abschnitt (10a) ein Vorverstärker (12a) angeordnet ist, welcher Signal leitend mit einem elektrischen Innenableiter (4a,20a) der Elektrode (4,20) verbunden ist.

20. Sensor (1) nach einem der Ansprüche 16 bis 19, **dadurch gekennzeichnet, dass** an der Oberfläche oder innerhalb der Leiterplatte (10) eine im wesentlichen vollflächige, elektrisch leitende Schicht (10b) angeordnet ist, welche vorzugsweise mit der Masse verbunden ist.

21. Sensor (1) nach einem der Ansprüche 16 bis 20, **dadurch gekennzeichnet, dass** die Elektrode (4;20) eine Längsrichtung (L) aufweist, dass die Leiterplatte (10) im wesentlichen senkrecht zu dieser Längsrichtung (L) verlaufend angeordnet ist, und dass die Leiterplatte (10) innerhalb der Länge der Elektrode (4;20) angeordnet ist.

22. Sensor (1) nach einem der Ansprüche 16 bis 21, **dadurch gekennzeichnet, dass** die Leiterplatte (10) eine Durchbrechung (10c) aufweist, dass eine der Messvorichtungen als elektrochemische Messvorrichtung ausgebildet ist, und dass die elektrochemische Messvorrichtung (19) durch die Durchbrechung (10c) verlaufend angeordnet ist.

23. Sensor (1) nach einem der Ansprüche 16 bis 22, **dadurch gekennzeichnet, dass** auf der Leiterplatte (10) störungsempfindliche elektronische Komponenten von den übrigen elektronischen Komponenten getrennt angeordnet sind.

24. Sensor (1) nach einem der Ansprüche 16 bis 23, **dadurch gekennzeichnet, dass** der Raum zwischen der Leiterplatte (10) und der Sensoroberfläche (1 b) ein wärmeleitendes Mittel (8) aufweist, wobei dieses wärmeleitende Mittel (8) vorzugsweise eingegossen ist.

25. Sensor (1) nach einem der Ansprüche 16 bis 24, **dadurch gekennzeichnet, dass** die bestückte Leiterplatte (10) zumindest teilweise von einer metallischen Schicht (16) umgeben ist.

26. Verfahren zum kombinierten Messen von zumindest zwei physiologischen Parametern wie Sauerstoff oder Kohlendioxid im Blut, indem mit einem Sensor (1) analoge Messwerte von im Sensor (1) angeordneten Messvorrichtungen (17, 19) erfasst werden, und indem ein Erwärmungssystem (18) im Sensor (1) geregelt erwärmt wird, **dadurch gekennzeichnet, dass** die analogen Messwerte im Sensor (1) in digitale Werte gewandelt und durch einen im Sensor (1) angeordneten digitalen Sensorsignalprozessor (13) verarbeitet werden, dass die vom Erwärmungssystem (18) erzeugte Temperatur durch den Sensorsignalprozessor (13) geregelt wird, und dass die verarbeiteten, digitalen Werte des Sensorsignalprozessors (13) in Form eines digitalen Signals über ein elektrisch leitendes Kabel (26a,26b) einer dem Sensor (1) nachfolgenden Vorrichtung (37, 44a) zugeführt werden.

27. Verfahren nach Anspruch 26, **dadurch gekennzeichnet, dass** im Sensor (1) Referenzdaten der Messvorrichtung (17, 19) gespeichert werden, dass die digitalen Werte entsprechend den Referenzdaten (49) kompensiert werden, und dass die derart kompensierten digitalen Werte der Signalauswertevorrichtung (37) zugeführt werden.

28. Verfahren nach einem der Ansprüche 26 oder 27, **dadurch gekennzeichnet, dass** durch den digitalen Sensorsignalprozessor (13) aus den Messwerten der Messvorrichtungen (17,19) der Wert der arteriellen Sauerstoffsättigung sowie der Wert des arteriellen Kohlendioxid-Partialdrucks berechnet wird.

29. Verfahren nach einem der Ansprüche 26 oder 28, **dadurch gekennzeichnet, dass** mit einem in der Signalauswertevorrichtung (37) angeordneten Prozessor (39) aus den digitalen Werten abhängig von den Eigenschaften der Messvorrichtung (17, 19) ein entsprechender physiologischer Parameter wie der Sauerstoff oder das Kohlendioxid im Blut oder die Pulsfrequenz berechnet wird.

30. Verfahren nach einem der Ansprüche 26 bis 29, **dadurch gekennzeichnet, dass** in einem Speicher des Sensors (1) Patientendaten gespeichert werden.

31. Verfahren nach einem der Ansprüche 26 bis 30, **dadurch gekennzeichnet, dass** zumindest Teile der in der Signalauswertevorrichtung (37) berechneten Werte der physiologischen Parameter wie Sauerstoff oder Kohlendioxid im Blut oder die Pulsfrequenz in einem Speicher des Sensors (1) gespeichert werden.

32. Verfahren nach Anspruch 31, **dadurch gekennzeichnet, dass** der Sensor (1) zeitlich nacheinander mit mehreren Signalauswertevorrichtungen (37) verbunden und wieder getrennt wird, und dass zumindest ein Teil der in der jeweiligen Signalauswertevorrichtung (37) berechneten Werte im Speicher des Sensors (1) gespeichert wird.

33. Verfahren nach Anspruch 32, **dadurch gekennzeichnet, dass** die jeweils mit dem Sensor (1) verbundene Signalauswertevorrichtung (37) zumindest ein Teil der im Speicher des Sensors (1) gespeicherten Daten übernimmt.

34. System zum Messen physiologischer Parameter umfassend einen Sensor (1) nach einem der Ansprüche 1 bis 25 sowie umfassend eine Signalauswertevorrichtung (37) mit einer digitalen Sensorschnittstelle (38) und einem Prozessor (39).

35. System nach Anspruch 34, **dadurch gekennzeichnet, dass** die digitale Sensorschnittstelle (38) eine digitale, signalübertragende Verbindung zum Sensor (1) ermöglicht, wobei der digitalen Sensorschnittstelle (38) der Prozessor (39) nachgeordnet ist, und wobei die Signalauswertevorrichtung (37) durch eine entsprechende Wahl der Software des Prozessors (39) zur Auswertung der Signale unterschiedlicher Sensoren (1) geeignet ist.

## Claims

1. Sensor (1) for the combined measurement of at least two physiological parameters such as oxygen or carbon dioxide in the blood, comprising measuring apparatus (17, 19), comprising a heating system (18) with an electric apparatus (6) as well as a temperature sensor (7), and also comprising an electrically conductive communication cable to a subsequent apparatus (37, 44a), wherein a digital sensor signal processor (13) is arranged in the sensor (1), which is connected in signal conducting manner to the measuring apparatus (17, 19) as well as to the heating apparatus (18), the sensor signal processor (13) is controlling the temperature of the sensor (1), the sensor signal processor (13) makes available a digital output signal, the sensor (1) comprises means (24) for a bidirectional, digital data transfer, and wherein the communication cable comprises a signal transmitting communication means (26) built as a two pole cable (26a, 26b), through which the digital output signal is transmitted to a subsequent apparatus (37, 44a) by bidirectional data transfer.

2. Sensor (1) in accordance with claim 1, **characterised in that** the heating apparatus (6) is built as an electric resistor and the electric resistor (6) is arranged within a heat conducting medium (8).

3. Sensor (1) in accordance with claim 2, **characterised in that** the heat conducting medium (8) consists of an electrically conductive epoxy resin.

4. Sensor (1) in accordance with claims 2 or 3, **characterised in that** the Sensor (1) has a sensor surface (1b) adapted to lay on a human body, and that the heat conducting medium (8) forms part of the sensor surface (1b).

5. Sensor (1) in accordance with one of the preceding claims, **characterised in that** the sensor (1) has a weight of less than 10 grams, and preferably a weight of less than 5 grams.

6. Sensor (1) in accordance with one of the preceding claims, comprising a data memory (23) in which a characteristic of at least one measuring apparatus (17, 19) can be stored.

7. Sensor (1) in accordance with one of the preceding claims for the combined measurement of the physiological parameters oxygen and carbon dioxide in the blood, comprising a measuring apparatus (17) to measure the arterial oxygen saturation as well as a measuring apparatus (19) to measure the arterial carbon dioxide partial pressure.

8. Sensor (1) in accordance with one of the preceding claim, comprising a measuring apparatus or an evaluating apparatus to measure the heart rate.

9. Sensor (1) in accordance with one of the preceding claims, **characterised in that** the sensor (1) has a sensor surface (1b) adapted to lay on an ear lobe.

10. Sensor (1) in accordance with one of the preceding claims, comprising at least one measuring apparatus (17, 19) from the following group:
- a measuring apparatus (19) for the measurement of the CO₂ content, in particular in accordance with Stow-Severinghaus,
- a measuring apparatus (19) for the measurement of the O₂ content, in particular in accordance with Clark,
- an optical measuring apparatus (17) with means for the pulse oximetric measurement of the arterial oxygen saturation comprising an LED (2; 2a, 2b) and a photodetector (3),
- a measuring apparatus for the measurement of the pulse frequency,
- a measuring apparatus for the measurement of the hematocrit,
- a measuring apparatus for the measurement of the blood pressure,
- a measuring apparatus for the measurement of components of the respiratory gas,
- a measuring apparatus for the measurement of the body temperature,
- a measuring apparatus for the measurement of the moisture content.

11. Sensor (1) in accordance with one of the preceding claims, **characterised in that** one of the measuring apparatus (17, 19) is formed as an electrochemical measuring apparatus, in particular for the measurement of the transcutaneous CO₂ partial pressure or of the transcutaneous O₂ partial pressure.

12. Sensor (1) in accordance with one of the preceding claims, **characterised in that** one of the measuring apparatus (17, 19) is formed as a semiconductor chip (19) and **in that** the semiconductor chip (19) permits the measurement of the concentration of a gas, such as CO₂, O₂, N or of a gas mixture such as an anaesthetic gas named Enflurane.

13. Sensor (1) in accordance with one of claims 1 to 11, **characterised in that** one of the measuring apparatus (17, 19) is formed as an optical measuring apparatus comprising a light source (2) and a photodetector (3).

14. Sensor (1) in accordance with claim 13, **characterised in that** one of the measuring apparatus (17, 19) includes a semiconductor chip (19) which permits the generation and/or measurement of light of different wavelengths.

15. Sensor (1) in accordance with one of the preceding claims, comprising a data memory (23), in which measured values and/or patient data can be stored.

16. Sensor (1) in accordance with one of the preceding claims, comprising an at least partly rigid circuit board (10) which is equipped with the electronic components (12, 13).

17. Sensor (1) in accordance with claim 16, **characterised in that** the circuit board (10) is equipped with an electrode (4; 20).

18. Sensor (1) in accordance with claim 16 or 17, **characterised in that** the circuit board (10) includes a flexible section (10a).

19. Sensor (1) in accordance with claim 18, **characterised in that** a preamplifier (12a) is arranged in the flexible section (10a) which is connected in signal-conducting manner to an inner electrical conductor (4a, 20a) of the electrode (4, 20).

20. Sensor (1) in accordance with on of claims 16 to 19, **characterised in that** a substantially full area, electrically conducting layer (10b) is arranged at the surface of or within the circuit board (10) and is preferably connected to ground.

21. Sensor (1) in accordance with one of claims 16 to 20, **characterised in that** the electrode (4; 20) has a longitudinal direction (L); **in that** the circuit board (10) is arranged extending substantially perpendicular to this longitudinal direction (L), and **in that** the circuit board (10) is arranged within the length of the electrode (4; 20).

22. Sensor (1) in accordance with one of claims 16 to 21, **characterised in that** the circuit board (10) has an aperture (10c), that one of the measuring apparatus is built as an electrochemical measuring apparatus, and **in that** the electrochemical measuring apparatus (19) is arranged extending through the aperture (10c).

23. Sensor (1) in accordance with one of claims 16 to 22, **characterised in that** electronic components sensitive to disturbance are arranged on the circuit board (10) separated from the remaining electronic components.

24. Sensor (1) in accordance with one of claims 16 to 23, **characterised in that** the space between the circuit board (10) and the sensor surface (1b) has a heat conducting medium (8), with the heat conducting medium (8) preferably being cast in place.

25. Sensor (1) in accordance with one of claim 16 to 24, **characterised in that** the circuit board assembly (10) is surrounded at least partly by a metallic layer (16).

26. Method for the combined measurement of at least two physiological parameters, such as oxygen or carbon dioxide in the blood, in which an analog measured value of a measuring apparatus (17, 19) is detected by a sensor (1), and in which a heating apparatus (18) arranged in the sensor (1) is heated under control, **characterised in that** the analog measured value is converted into digital values in the sensor (1) and processed by a digital sensor signal processor (13) arranged in the sensor (1), that the temperature produced by the heating apparatus (18) is controlled by the sensor signal processor (13), and **in that** the processed digital values of the sensor signal processor (13) are supplied as a digital signal through an electric conductive cable (26a,26b) to a device (37, 44a) arranged subsequent to the sensor (1).

27. Method in accordance with claim 26, **characterised in that** reference data of the measuring apparatus (17, 19) are stored in the sensor (1), **in that** the digital values corresponding to the reference data (49) are compensated, and **in that** the so compensated digital values are supplied to the signal evaluation device (37).

28. Method in accordance with claim 26 or 27, **characterised in that** the digital sensor signal processor (13) calculates from the measured values of the measuring apparatus (17, 19) the value of the arterial oxygen saturation as well as the value of the carbon dioxide partial pressure.

29. Method in accordance with one of claims 26 or 28, **characterised in that** a corresponding physiological parameter, such as the oxygen or the carbon dioxide in the blood or the pulse frequency, is calculated from the digital values in dependence on the characteristics of the measuring apparatus (17, 19) using a processor (39) arranged in the signal evaluation device (37).

30. Method in accordance with one of claims 26 to 29, **characterised in that** patient data are stored in a memory of the sensor (1).

31. Method in accordance with one of claims 26 to 30, **characterised in that** at least parts of the values of the physiological parameters, such as oxygen or carbon dioxide in the blood or the pulse frequency, calculated in the signal evaluation device (37) are stored in a memory of the sensor (1).

32. Method in accordance with claim 31, **characterised in that** the sensor (1) is connected with a plurality of signal evaluation devices (37) timewise one after the other and separated again, and **in that** at least a part of the values calculated in the respective signal evaluation device (37) are stored in the memory of the sensor (1).

33. Method in accordance with claim 32, **characterised in that** the signal evaluation device (37) respectively connected to the sensor (1) takes over at least a part of the data stored in the memory of the sensor (1).

34. System for the measurement of physiological parameters comprising a sensor (1) in accordance with one of claims 1 to 25, and also comprising a signal evaluation device (37) with a digital sensor interface (38) and a processor (39).

35. System in accordance with claim 34, **characterised in that** the digital sensor interface (38) enables a digital signal transmitting connection to the sensor (1), with the digital sensor interface (38) being arranged after the processor (39) and with the signal evaluation device (37) being suited for the evaluation of the signals of different sensors (1) through a corresponding choice of the software of the processor (39).

## Revendications

1. Capteur (1) pour la mesure combinée d'au moins deux paramètres physiologiques tels que l'oxygène ou le dioxyde de carbone dans le sang, comprenant des dispositifs de mesure (17, 19), comprenant un système de chauffage (18) avec un dispositif de chauffage (6) ainsi que comprenant un câble de connexion électroconducteur allant à un dispositif (37, 44a) suivant, dans lequel capteur (1) est disposé un processeur de signaux numériques de capteur (13) qui est en liaison conductrice de signaux avec les dispositifs de mesure (17, 19) ainsi que le système de chauffage (18), le processeur de signaux numériques de capteur (13) régule la température du capteur (1), le processeur de signaux numériques de capteur (13) met à disposition un signal de sortie numérique, le capteur (1) présente des moyens (24) pour un échange de données bidirectionnel numérique et dans lequel le câble de connexion présente un moyen de connexion (26) permettant la transmission de signaux réalisé sous la forme d'un câble (26a, 26b) au moins bipolaire par lequel s'effectue la transmission des signaux de sortie numériques au moyen d'un échange de données bidirectionnel vers le dispositif (37, 44a) suivant.

2. Capteur (1) selon la revendication 1, **caractérisé par le fait que** le dispositif de chauffage (6) est réalisé sous la forme d'une résistance électrique et que la résistance électrique (6) est entourée par une masse coulée thermoconductrice (8).

3. Capteur (1) selon la revendication 2, **caractérisé par le fait que** la masse coulée thermoconductrice (8) est réalisée en résine époxy électriquement isolante.

4. Capteur (1) selon la revendication 2 ou 3, **caractérisé par le fait que** le capteur (1) présente une surface de capteur (1b) destinée à être appliquée sur le corps humain et que la masse coulée thermoconductrice (8) forme une partie de la surface de capteur (1b).

5. Capteur (1) selon l'une des revendications précédentes, **caractérisé par le fait que** le capteur (1) présente un poids de moins de 10 grammes et de préférence un poids de moins de 5 grammes.

6. Capteur (1) selon l'une des revendications précédentes, comprenant une mémoire de données (23) dans laquelle une courbe caractéristique d'au moins un dispositif de mesure (17, 19) est mémorisable.

7. Capteur (1) selon l'une des revendications précédentes pour la mesure combinée des paramètres physiologiques oxygène et dioxyde de carbone dans le sang, comprenant un dispositif de mesure (17) pour la mesure de la saturation artérielle en oxygène ainsi qu'un dispositif de mesure (19) pour la mesure de la pression partielle artérielle en dioxyde de carbone.

8. Capteur (1) selon l'une des revendications précédentes, comprenant un dispositif de mesure ou d'évaluation pour la saisie de la fréquence du pouls.

9. Capteur (1) selon l'une des revendications précédentes, **caractérisé par le fait que** le capteur (1) présente une surface d'appui de capteur (1b) destinée à être appliquée sur un lobe de l'oreille.

10. Capteur (1) selon l'une des revendications précédentes, comprenant un dispositif de mesure (17, 19) du groupe suivant :
- un dispositif de mesure (19) pour la mesure de la concentration en CO₂, en particulier selon Stow-Severinghaus,
- un dispositif de mesure (19) pour la mesure de la concentration en O₂, en particulier selon Clark,
- un dispositif de mesure optique (17) avec des moyens pour la mesure pulsoxymétrique de la saturation artérielle en oxygène comprenant une DEL (2 ; 2a, 2b) et un photodétecteur (3),
- un dispositif de mesure pour la mesure de la fréquence du pouls,
- un dispositif de mesure pour la mesure de l'hématocrite,
- un dispositif de mesure pour la mesure de la pression artérielle,
- un dispositif de mesure pour la mesure des composants du gaz respiratoire,
- un dispositif de mesure pour la mesure de la température corporelle,
- un dispositif de mesure pour la mesure de l'humidité.

11. Capteur (1) selon l'une des revendications précédentes, **caractérisé par le fait que** l'un des dispositifs de mesure (17, 19) est réalisé sous la forme d'un dispositif de mesure électrochimique, en particulier pour la mesure de la pression partielle transcutanée en CO₂ ou de la pression partielle transcutanée en O₂.

12. Capteur (1) selon l'une des revendications précédentes, **caractérisé par le fait que** l'un des dispositifs de mesure (17, 19) est réalisé sous la forme d'une puce à semi-conducteurs (19) et que la puce à semi-conducteurs (19) permet de mesurer la concentration d'un gaz tel que CO₂, O₂, N ou d'un mélange de gaz tel qu'un gaz anesthésiant désigné par enflurane.

13. Capteur (1) selon l'une des revendications 1 à 11, **caractérisé par le fait que** l'un des dispositifs de mesure (17, 19) est réalisé sous la forme d'un dispositif de mesure optique comprenant une source de lumière (2) et un photodétecteur (3).

14. Capteur (1) selon la revendication 13, **caractérisé par le fait que** l'un des dispositifs de mesure (17, 19) comprend une puce à semi-conducteurs (19) qui permet de générer et/ou de mesurer de la lumière de longueur d'onde différente.

15. Capteur (1) selon l'une des revendications précédentes, comprenant une mémoire de données (23) dans laquelle des valeurs de mesure et/ou des données de patient sont mémorisables.

16. Capteur (1) selon l'une des revendications précédentes, comprenant une carte à circuit imprimé (10) au moins partiellement rigide qui est équipée de composants électroniques (12, 13).

17. Capteur (1) selon la revendication 16, **caractérisé par le fait que** la carte à circuit imprimé (10) est équipée d'une électrode (4 ; 20).

18. Capteur (1) selon la revendication 16 ou 17, **caractérisé par le fait que** la carte à circuit imprimé (10) comprend une partie flexible (10a).

19. Capteur (1) selon la revendication 18, **caractérisé par le fait que** dans la partie flexible (10a) est disposé un préamplificateur (12a) qui est en liaison conductrice de signaux avec un conducteur électrique intérieur (4a, 20a) de l'électrode (4, 20).

20. Capteur (1) selon l'une des revendications 16 à 19, **caractérisé par le fait qu'**une couche électroconductrice (10b) qui est de préférence reliée à la masse est disposée à la surface ou à l'intérieur de la carte à circuit imprimé (10), essentiellement sur toute la surface.

21. Capteur (1) selon l'une des revendications 16 à 20, **caractérisé par le fait que** l'électrode (4, 20) présente une direction longitudinale (L), que la carte à circuit imprimé (10) est disposée essentiellement perpendiculairement par rapport à cette direction longitudinale (L) et que la carte à circuit imprimé (10) est disposée à l'intérieur de la longueur de l'électrode (4, 20).

22. Capteur (1) selon l'une des revendications 16 à 21, **caractérisé par le fait que** la carte à circuit imprimé (10) présente une ouverture (10c), que l'un des dispositifs de mesure est réalisé sous la forme d'un dispositif de mesure électrochimique et que le dispositif de mesure électrochimique (19) s'étend à travers l'ouverture (10c).

23. Capteur (1) selon l'une des revendications 16 à 22, **caractérisé par le fait que** sur la carte à circuit imprimé (10) les composants électroniques sensibles aux perturbations sont séparés des autres composants électroniques.

24. Capteur (1) selon l'une des revendications 16 à 23, **caractérisé par le fait que** l'espace entre la carte à circuit imprimé (10) et la surface de capteur (1b) présente un moyen thermoconducteur (8), ce moyen thermoconducteur (8) étant de préférence coulé.

25. Capteur (1) selon l'une des revendications 16 à 24, **caractérisé par le fait que** la carte à circuit imprimé (10) équipée est entourée au moins partiellement par une couche métallique (16).

26. Procédé pour la mesure combinée d'au moins deux paramètres physiologiques tels que l'oxygène ou le dioxyde de carbone dans le sang, selon lequel des valeurs de mesure analogiques sont saisies avec un capteur (1) par des dispositifs de mesure (17, 19) disposés dans le capteur (1) et selon lequel un système de chauffage (18) dans le capteur (1) est réchauffé de manière régulée, **caractérisé par le fait que** les valeurs de mesure analogiques sont converties en valeurs numériques dans le capteur (1) et traitées par un processeur de signaux numériques de capteur (13) disposé dans le capteur (1), que la température générée par le système de chauffage (18) est régulée par le processeur de signaux numériques de capteur (13) et que les valeurs numériques traitées du processeur de signaux numériques de capteur (13) sont amenées sous la forme d'un signal numérique à un dispositif (37, 44a) qui suit le capteur (1) via un câble électroconducteur (26a, 26b).

27. Procédé selon la revendication 26, **caractérisé par le fait que** des données de référence du dispositif de mesure (17, 19) sont mémorisées dans le capteur (1), que les valeurs numériques sont compensées en fonction des données de référence (49) et que les valeurs numériques ainsi compensées sont amenées au dispositif d'évaluation de signaux (37).

28. Procédé selon l'une des revendications 26 ou 27, **caractérisé par le fait que** le processeur de signaux numériques de capteur (13) calcule à partir des valeurs de mesure des dispositifs de mesure (17, 19) la valeur de la saturation artérielle en oxygène ainsi que la valeur de la pression partielle artérielle en dioxyde de carbone.

29. Procédé selon l'une des revendications 26 ou 28, **caractérisé par le fait que** l'on calcule avec un processeur (39) disposé dans le dispositif d'évaluation de signaux (37) à partir des valeurs numériques et en fonction des propriétés du dispositif de mesure (17, 19) un paramètre physiologique correspondant tel que l'oxygène ou le dioxyde de carbone dans le sang ou la fréquence du pouls.

30. Procédé selon l'une des revendications 26 à 29, **caractérisé par le fait que** des données de patient sont enregistrées dans une mémoire du capteur (1).

31. Procédé selon l'une des revendications 26 à 30, **caractérisé par le fait qu'**au moins des parties des valeurs de paramètres physiologiques tels que l'oxygène ou le dioxyde de carbone dans le sang ou la fréquence du pouls calculées dans le dispositif d'évaluation de signaux (37) sont enregistrées dans une mémoire du capteur (1).

32. Procédé selon la revendication 31, **caractérisé par le fait que** le capteur (1) est relié et de nouveau séparé successivement dans le temps à plusieurs dispositifs d'évaluation de signaux (37) et qu'au moins une partie des valeurs calculées dans le dispositif d'évaluation de signaux (37) respectif est enregistrée dans la mémoire du capteur (1).

33. Procédé selon la revendication 32, **caractérisé par le fait que** le dispositif d'évaluation de signaux (37) respectivement relié au capteur (1) prend en charge au moins une partie des données enregistrées dans la mémoire du capteur (1).

34. Système pour la mesure de paramètres physiologiques comprenant un capteur (1) selon l'une des revendications 1 à 25 ainsi que comprenant un dispositif d'évaluation de signaux (37) avec une interface de capteur numérique (38) et un processeur (39).

35. Système selon la revendication 34, **caractérisé par le fait que** l'interface de capteur numérique (38) permet une liaison numérique pour la transmission de signaux avec le capteur (1), dans lequel l'interface de capteur numérique (38) est placée à la suite du processeur (39) et dans lequel le dispositif d'évaluation de signaux (37) est adapté à l'évaluation des signaux de différents capteurs (1) par un choix correspondant du logiciel du processeur (39).
